(19) Europäisches Patentamt
European Patent Office
Office européen des brevets

(11) Veröffentlichungsnummer: **0 003 484**
**B1**

(12) # EUROPÄISCHE PATENTSCHRIFT

(45) Veröffentlichungstag der Patentschrift: **14.10.81**

(51) Int. Cl.$^3$: **C 07 D 311/72**

(21) Anmeldenummer: **78101866.8**

(22) Anmeldetag: **29.12.78**

(54) **Verfahren zur Herstellung von Chromanderivaten.**

(30) Priorität: **30.01.78 US 873184**

(43) Veröffentlichungstag der Anmeldung:
**22.08.79 Patentblatt 79/17**

(45) Bekanntmachung des Hinweises auf die Patenterteilung:
**14.10.81 Patentblatt 81/41**

(84) Benannte Vertragsstaaten:
**BE CH DE FR GB IT NL**

(56) Entgegenhaltungen:
**FR - A - 2 255 299**

(73) Patentinhaber: **F. HOFFMANN-LA ROCHE & CO. Aktiengesellschaft**
**CH-4002 Basel (CH)**

(72) Erfinder: **Cohen, Noal**
**19 Euclid Place**
**Montclair, N.J. (US)**

(74) Vertreter: **Cottong, Norbert A. et al,**
**124 Grenzacherstrasse P.O. Box 601**
**CH-4002 Basel (CH)**

**EP 0 003 484 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Verfahren zur Herstellung von Chromanderivaten

Die vorliegende Erfindung betrifft ein Verfahren zur Herstellung von Chromanderivaten der allgemeinen Formel

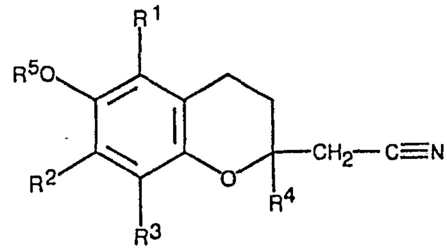

I

worin $R^1$, $R^2$, $R^3$ Wasserstoff oer niederes Alkyl, $R^4$ niederes Alkyl und $R^5$ Wasserstoff oder eine durch Hydrolyse abspaltbare Säureschutzgruppe darstellen,
sowie, gewünschtenfalls, deren Ueberführung in Verbindungen der allgemeinen Formel

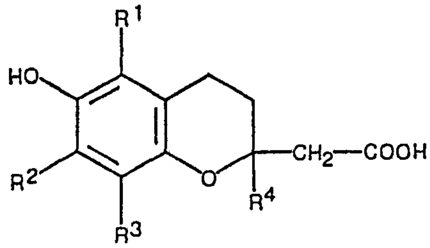

II

worin $R^1$, $R^2$, $R^3$ und $R^4$ die obige Bedeutung haben.

Die Verbindungen der obigen Formel II sowie deren Herstellung sind beispielsweise aus der französischen Patentpublikation No. 2 255 299 bekannt. Diese Verbindungen sind bekannte Antioxidantien und diejenige Verbindung, worin $R^1$, $R^2$, $R^3$ und $R^4$ Methylgruppen darstellen, ist ein bekanntes Zwischenprodukt in der Herstellung von Vitamin E.

Im Rahmen der vorliegenden Erfindung bedeutet der Ausdruck "niederes Alkyl" sowohl geradkettige als auch verzweigte, gesättigte Kohlenwasserstoffreste mit 1—7 Kohlenstoffatomen wie etwa Methyl, Aethyl, Propyl, Isopropyl und dergleichen. Weiterhin bedeutet der Ausdruck "Halogen" alle vier Halogenatome, nämlich Brom, Chlor, Fluor und Jod. Der Ausdruck "Alkalimetall" umfasst Natrium Kalium und Lithium.

Weitherhin bedeutet im Rahmen dieser Erfindung der Ausdruck "Aryl" sowohl einkernige aromatische Kohlenwasserstoffgruppen, wie Phenyl, welche unsubstituiert oder in einer oder mehreren Stellungen mit niederen Alkylgruppen substituiert sein können als auch mehrkernige Kohlenwasserstoffgruppen, wie Naphthyl, Anthryl, Phenanthryl, Azulyl und dergleichen, welche ebenfalls unsubstituiert oder mit einer oder mehreren der vorgehend erwähnten Gruppen substituiert sein können. Bevorzugte Arylgruppen sind substitutierte and unsubstituierte einkernige Arylgruppen, insbesondere Phenyl und Tolyl. Der Ausdruck "Aryl- niederes Alkyl" bedeutet Gruppen worin der Aryl- und niedere Alkylrest wie vorgehend definiert sind, insbesondere Benzyl. Der Ausdruck "Arylcarbonsäure" bedeutet Säuren worin die Arylgruppe wie vorhergehend definiert ist. Die bevorzugte Arylcarbonsäure ist die Benzoesäure.

Weiterhin bedeutet im Rahmen der vorliegenden Erfindung der Ausdruck "durch Hydrolyse abspaltbare Säureschutzgruppe" jegliche herkömmliche organische Säureschutzgruppe, welche durch Hydrolyse abgespalten werden kann. Die hierzu bevorzugten organischen Sauren sind die niederen Alkancarbonsäuren mit 2—7 Kohlenstoffatomen wie Essigsäure, Propionsäure und dergleichen, sowie auch die Arylcarbonsäuren wie Benzoesäure und dergleichen.

Das erfindungsgemässe Verfahren ist dadurch gekennzeichnet, dass man eine Verbindung der allgemeinen Formel

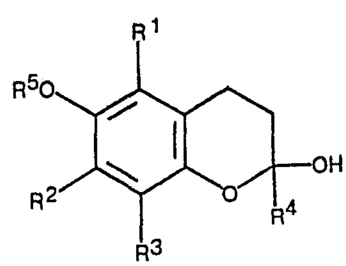

III



stituiert oder mit einer oder mehreren der vorgehend erwähnten Gruppen substituiert können. Be-

worin $R^1$, $R^2$, $R^3$, $R^4$ und $R^5$ die obige Bedeutung haben,
mit Acetonitril in Gegenwart einer starken Base umsetzt, oder dass man eine Verbindung der allgemeinen Formel

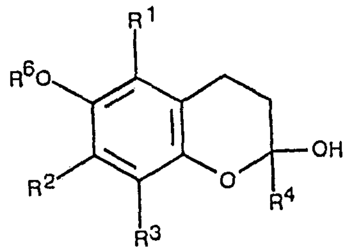

IIIA

worin $R^1$, $R^2$, $R^3$ und $R^4$ die obige Bedeutung haben, und $R^6$ eine hydrolytisch abspaltbare Säureschutzgruppe darstellt,
mit Cyanessigsäure in einer organische Aminbase in Gegenwart einer Verbindung der allgemeinen Formel

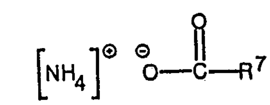

IV

worin $R^7$ niederes Alkyl oder Aryl darstellt, umsetzt und dass man, gewünschtenfalls, eine so erhaltene Verbindung der Formel I in eine Verbindung der Formel II überführt.

Bei der Umsetzung einer Verbindung der Formel III mit Acetonitril kann jede beliebige starke Base verwendet werden. Von den bevorzugten Basen können insbesondere Alkalimetallhydroxide, wie Natrium-oder Kaliumhydroxid genannt werden. Bei der Umsetzung mit einer solchen Base wird auch der Rest $R^5$, sofern dieser eine Schutzgruppe darstellt, abgespalten und man erhält eine Verbindung der allgemeinen Formel

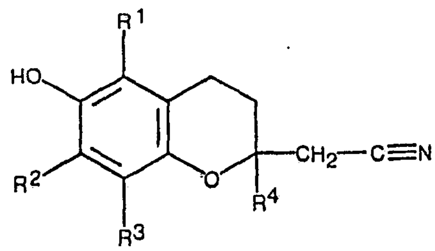

IA

worin $R^1$, $R^2$, $R^3$ und $R^4$ die obige Bedeutung haben. Das Acetonitril kann als Reaktionsmedium verwendet werden. Deshalb ist es bevorzugt, diese Reaktion in einem Ueberschuss an Acetonitril durchzuführen. Weiterhin wird diese Reaktion normalerweise bei Rückflusstemperatur des Reaktionsgemisches durchgeführt.

Bei der Umsetzung einer Verbindung der Formel III—A mit Cyanessigsäure in Gegenwart einer Verbindung der Formel IV, erhält man eine Verbindung der allgemeinen formel

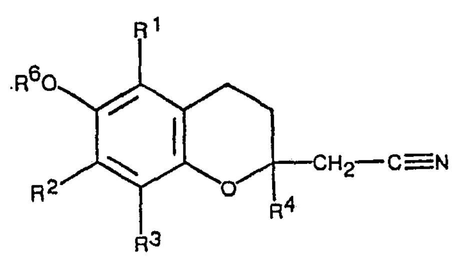

IB

worin $R^1$, $R^2$, $R^3$, $R^4$ und $R^6$ die obige Bedeutung haben. Diese Reaktion erfolgt in Gegenwart eines organischen Lösungsmittels, wobei jegliches inerte organische Lösungsmittel mit einem Sdp. oberhalb 80°C verwendet werden kann. Bevorzugte derartige Lösungsmittel sind beispielsweise aromatische Kohlenwasserstoffe, wie etwa Toluol, Benzol, Xylol usw. Normalerweise erfolgt diese Reaktion unter den Bedingungen einer Knoevenagel-Kondensation [siehe z.B. G. Jones, Organic Reactions, *15*, 204 (1967)]. Weiterhin erfolgt die Reaktion gewöhnlich in Gegenwart einer organischen Aminbase, wie etwa Pyridin. Es können jedoch auch andere Amine, wie 'Tri(niederalkyl) amine, beispielsweise Triäthylamin, Trimethylamin und dergleichen verwendet werden. Bei dieser Reaktion liegt die Temperatur zwischen etwa 80°C und etwa 150°C. Eine so erhaltene Verbindung der Formel I—B kann direkt in eine Verbindung der Formel II übergeführt werden durch Behandlung mit einer starken anorganischen Base wie einem Alkalimetallhydroxid, beispielsweise Kaliumhydroxid, Natriumhydroxid usw. Die Durchführung dieser Reaktion erfolgt gewöhnlich bei Temperaturen von etwa 100°C bis etwa 200°C. Gewöhnlich wird diese Reaktion in einem hochsiedenden organischen Lösungsmittel durchgeführt wie etwa Aethylenglycol oder auch einem anderen vorhergehend genannten organischen Lösungsmittel.

Eine erhaltene Verbindung der Formel I—B kann, andererseits, auch in eine Verbindung der Formel II via eine Verbindung der Formel I—A übergeführt werden. Hierbei wird die erhaltene Verbindung mit einem Alkalimetallcarbonat oder Alkalimetallbicarbonat bei einer Temperatur von etwa 20°C bis 65°C behandelt. Bei dieser Reaktion kann jegliches inerte organische Lösungsmittel sowie auch Wasser verwendet werden. Gewöhnlich erfolgt die Reaktion bei Temperaturen zwischen etwa 20°C und 65°C. Eine so erhaltene Verbindung der Formel I—A kann dann in eine Verbindung der Formel II übergeführt werden durch Behandlung mit einer starken Base wie einem Alkalimetallhydroxid unter den vorhergehend für die direkte Umsetzung einer Verbindung der Formel I—B zu einer Verbindung der Formel II angegebenen Bedingungen.

Die nachfolgenden Beispiele sind als solche zu verstehen und sollen die Erfindung in keiner Weise beschränken. In diesen Beispielen wurden sämtliche Reaktionen unter einer Argonatmosphäre durchgeführt. Das "übliche Aufarbeiten" umfasste drei Extraktionen mit dem speziell erwähnten Lösungsmittel. Die organischen Extrakte wurden mit Wasser und Sole gewaschen, dann über Magnesiumsulfat getrocknet, filtriert und bei 40—50° C am Rotationsverdampfer eingeengt. Der Rückstandwurde weiter unter Hochvakuum getrocknet. Kolonnenchromatographie erfolgte unter Verwendung von Silicalgel, 0,063—0,2 mm. Der in den Beispielen verwendete Aether war Diäthyläther. Alle Temperaturen sind in °C.

Beispiel 1

rac.-3,4-Dihydro-6-hydroxy-2,5,7,8-tetramethyl-2H-1-benzopyran-2-acetonitril.

Ein Gemisch von 9,0 g (40,5 mMol) 2,6-Dihydroxy-2,5,7,8-tetramethylchroman und 4,54 g (69 mMol) 85%-ige KOH in 61 ml Acetonitril wurde rasch während 4 Stunden am Rückfluss gerührt. Das entstandene dunkelgefärbte Gemisch, welches aus zwei Phasen bestand, wurde in einem Eisbad gekühlt und tropfenweise unter Rühren mit 25 ml 3N wässriger HCl versetzt, wobei das Gemisch eine hellere Farbe annahm. Dieses Gemisch wurde in 1N wässrige HCl gegossen und unter Verwendung von Aethylacetat in üblicher Weise aufgearbeitet (die organischen Extrakte wurden zusätzlich mit gesättigter, wässriger $NaHCO_3$-Lösung gewaschen). Man erhielt 14.8 g rac.3,4-Dihydro-6-hydroxy-2,5,7,8-tetramethyl-2H-1-benzopyran-2-acetonitril; Schmelzpunkt 162°—163°.

Beispiel 2

rac.6-Acetyloxy-3,4-dihydro-2,5,7,8-tetramethyl-2H-1-benzopyran-2-acetonitril

Eine Lösung von 26,4 g (0,1 Mol) rac.2 - Hydroxy - 6 - acetoxy - 3,4 - dihydro - 2,5,7,8 - tetramethyl - 2H - 1 - benzopyran, 8,5 g (0,1 Mol) Cyanessigsäure und 300 mg Ammoniumacetat in 11 ml Pyridin und 20 ml Toluol wurde unter Rühren und Wasserentfernung während 18 Stunden am Rückfluss erhitzt. Das Reaktionsgemisch wurde auf Raumtemperatur abgekühlt und filtriert. Das Filtrat wurde mit Aether verdünnt und zweimal mit 3N wässriger HCl und je einmal mit gesättigter wässriger $NaHCO_3$ und Sole gewaschen. Die organische Lösung wurde in üblicher Weise aufgearbeitet und man erhielt 24,1 g eines gelben Feststoffes. Dieses Material wurde in 110 ml heissem Aether gelöst und dann auf 0°C abgekühlt. Der entstandene Niederschlag wurde abfiltriert. Das Filtrat wurde dann in einem Rotationsverdampfer eingeengt und dann an 250 mg Silicagel chromatographiert. Durch Elution mit 2:1 Volumenteilen und 1:1 Volumenteilen Hexan/Aether erhielt man 8,44 g rac.6-acetyloxy-3,4-dihydro-2,5,7,8-tetramethyl-2H-1-benzopyran-2-acetonitril in Form eines weissen Feststoffes. Eine Probe dieses Materials wurde aus Aethylacetat umkristallisiert und man erhielt weisse Kristalle mit einem Schmelzpunkt von 122—123°C.

Beispiel 3

rac.3,4-Dihydro-6-hydroxy-2,5,7,8-tetramethyl-2H-1-benzopyran-2-acetonitril

Ein Gemisch von 581 mg (2,02 mMl) rac.6 - Acetoloxy - 3,4 - dihydro - 2,5,7,8 - tetramethyl - 2H - 1 - benzopyran - 2 - acetonitril und 418 mg (3,03 mMol) $K_2CO_3$ in 17 ml Methanol und 3 ml Wasser wurde bei Raumtemperatur während 64 Stunden gerührt. Das erhaltene Gemisch wurde in Wasser gegossen und mit Aether in üblicher Weise aufgearbeitet und man erhielt 480 mg eines gelbbraunen Feststoffes. Dieses Material wurde mit kaltem Aether behandelt und man erhielt 340 mg eines ebenfalls gelbfarbenen Feststoffes. Eine Probe dieses Materials wurde aus Toluol umkristallisiert und man erhielt reines rac.3,4 - Dihydro - 6 - hydroxy - 2,5,7,8 - tetramethyl - 2H - 1 - benzopyran - 2 - acetonitril mit einem Schmelzpunkt von 162—163°C.

Beispiel 4

rac.(6-Hydroxy-3,4-dihydro-2,5,7,8-tetramethyl-2H-1-benzopyran-2-yl)-essigsäure

Ein Gemisch von 14,8g rac.3,4 - Dihydro - 6 - hydroxy - 2,5,7,8 - tetramethyl - 2H - 1 - benzopyran - 2 - acetonitril (hergestellt gemäss Beispiel 1 oder 3), 10,2 (155 mMol) 85%ige KOH, 9 ml Wasser und 83 ml Aethylenglycol wurde unter Rühren während 17,5 Stunden am Rückfluss erhitzt.

Die erhaltene Lösung wurde dann auf Raumtemperatur abgekühlt und mit 250 ml Wasser verdünnt. Der pH der Lösung wurde auf 8 mittels 3N wässriger HCl eingestellt und das erhaltene Gemisch mit 3 Portionen Aether extrahiert (die Aetherextrakte wurden verworfen). Die wässrige Phase wurde weiter mit Wasser verdünnt und durch tropfenweise Zugabe von 3N wässriger HCl angesäuert. Der erhaltene Niederschlag wurde abfiltriert, mit Wasser gewaschen und dann während 24 Stunden bei Raumtemperatur unter Hochvakuum getrocknet. Man erhielt 8,05 g rac.(6 - Hydroxy - 3,4 - dihydro - 2,5,7,8 - tetramethyl - 2H - 1 - benzopyran - 2 - yl) - essigsäure mit einem Schmelzpunkt von 169—172°C.

Beispiel 5

rac.(6-Hydroxy-3,4-dihydro-2,5,7,8-tetramethyl-2H-1-benzopyran-2-yl)-essigsäure

Ein Gemisch von 1,0 g (3,48 mMol) rac.6 - Acetyloxy - 3,4 - dihydro - 2,5,7,8 - tetramethyl - 2H - 1 - benzopyran - 2 - acetonitril, 1,0 g (15,2 mMol) 84%ige KOH, 1 ml Wasser und 8 ml Aethylenglycol wurde während 16 Stunden am Rückfluss gerührt. Das Reaktionsgemisch wurde dann auf Raumtemperatur abgekühlt und in Wasser gegossen. Der pH der Lösung wurde mit 1N wässriger HCl auf 8 eingestellt. Das erhaltene Gemisch wurde mit 2 Portionen Aether extrahiert (die Aetherextrakte wurden verworfen). Die wässrige Phase wurde mit 3N wässriger HCl angesäuert und mit Aether in üblicher Weise aufgearbeitet. Man erhielt 756 mg rac.(6 - Hydroxy - 3,4 - dihydro - 2,5,7,8 - tetramethyl - 2H - 1 - benzopyran - 2 - yl) - essigsäure mit einem Schmelzpunkt von 169—172°C.

**Patentansprüche**

1. Verfahren zur Herstellung von Chromanderivaten der allgemeinen Formeln I und II

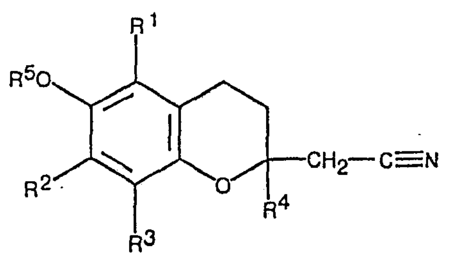 I

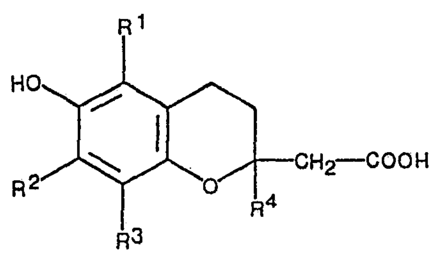 II

worin $R^1$, $R^2$, $R^3$ Wasserstoff oder niederes Alkyl, $R^4$ niederes Alkyl und $R^5$ Wasserstoff oder eine durch Hydrolyse abspaltbare Säureschutzgruppe darstellen,

dadurch gekennzeichnet, dass man eine Verbindung der allgemeinen Formel III

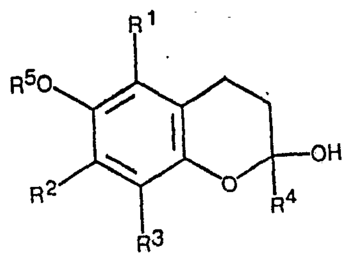 III

worin $R^1$, $R^2$, $R^3$, $R^4$ und $R^5$ die obige Bedeutung haben, mit Acetonitril in Gegenwart einer starken Base umsetzt, oder dass man eine Verbindung der allgemeinen Formel IIIA

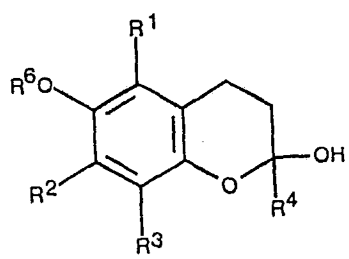 IIIA

worin $R^1$, $R^2$, $R^3$ und $R^4$ die obige Bedeutung haben und $R^6$ eine hydrolytisch abspaltbare Säureschutzgrupper darstellt,

mit Cyanessigsäure in einer organischen Aminbase in Gegenwart einer Verbindung der allgemeinen Formel

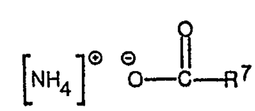 IV

worin $R^7$ niederes Alkyl oder Aryl darstellt, umsetzt und dass man gewünschtenfalls eine so erhaltene Verbindung der allgemeinen Formel I in eine Verbindung der allgemeinen Formel II überführt.

2. Verfahren gemäss Anspruch 1, dadurch gekennzeichnet, dass man als starke Base ein Alkalimetallhydroxid verwendet.

3. Verfahren gemäss Anspruch 1, dadurch gekennzeichnet, dass man als organische Aminbase Pyridin verwendet.

**Claims**

1. Process for the manufacture of chromane derivatives of the general formulae I and II

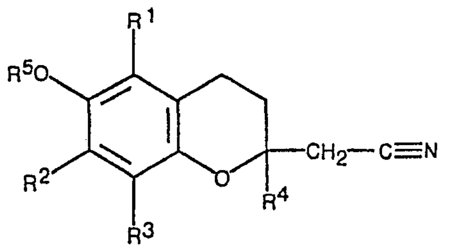 I

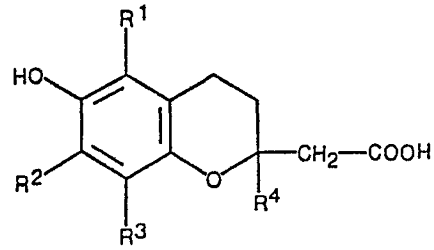 II

wherein $R^1$, $R^2$, $R^3$ represent hydrogen or lower alkyl, $R^4$ represents lower alkyl and $R^5$ represents hydrogen or an acid protecting group cleavable by hydrolysis,

characterised by reacting a compound of the general formula III

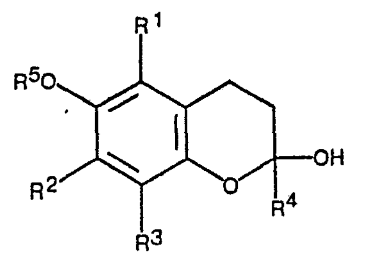 III

wherein $R^1$, $R^2$, $R^3$, $R^4$ and $R^5$ have the above significance,

with acetonitrile in the presence of a strong base, or by reacting a compound of the general formula IIIA

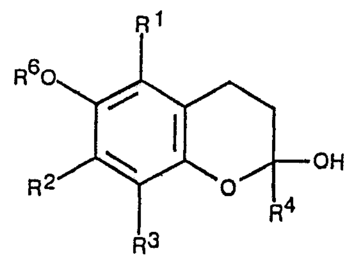 IIIA

wherein $R^1$, $R^2$, $R^3$ and $R^4$ have the above significance and $R^6$ represents a hydrolytically cleavable acid protecting group,

with cyanoacetic acid in an organic amine base in the presence of a compound of the general formula

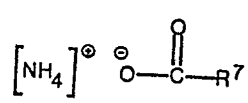 IV

wherein $R^7$ represents lower alkyl or aryl,

and, if desired, converting a thus-obtained compound of the general formula I into a compound of the general formula II.

2. Process in accordance with claim 1, characterised in that an alkali metal hydroxide is used as the strong base.

3. Process in accordance with claim 1, characterised in that pyridine is used as the organic amine base.

**Revendications**

1. Procédé de préparation de dérivés du chromane répondant aux formules générales I et II

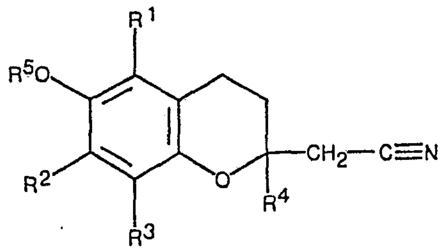 I , 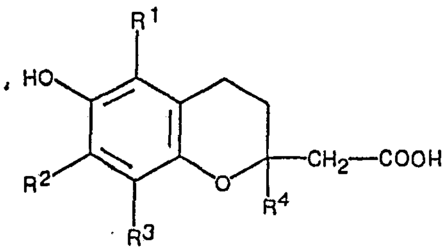 II

dans lesquelles $R^1$, $R^2$, $R^3$ représentent des atomes d'hydrogène ou des groupes alkyles inférieurs, $R^4$ représente un groupe alkyle inférieur et $R^5$ l'hydrogène ou un groupe protecteur acide éliminable par hydrolyse, caractérisé en ce que l'on fait réagir un composé de formule générale III

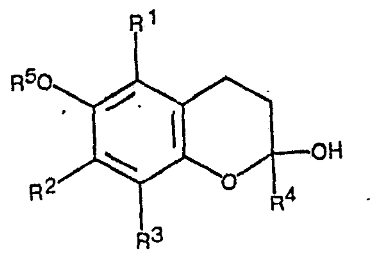

III

dans laquelle $R^1$, $R^2$, $R^3$ et $R^4$ et $R^5$ ont les significations indiquées ci-dessus, avec l'acétonitrile en présence d'une base forte,
ou bien en ce que l'on fait réagir un composé de formule générale III—A

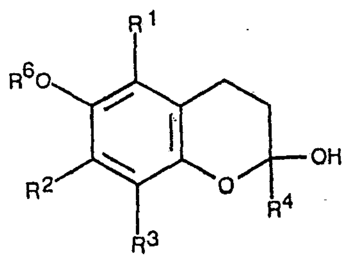

IIIA

dans laquelle $R^1$, $R^2$, $R^3$ et $R^4$ ont les significations indiquées ci-dessus et $R^6$ représente un groupe protecteur acide éliminable par hydrolyse,
avec l'acide cyanétique dans une base aminée organique en présence d'un composé de formule générale

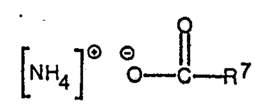

IV

dans laquelle $R^7$ représente un groupe alkyle inférieur ou aryle,
et si on le désire, ou convertit un composé de formule générale I ainsi obtenu en un composé de formule générale II.

2. Procédé selon la revendication 1, caractérisé en ce que l'on utilise en tant que base forte un hydroxyde de métal alcalin.

3. Procédé selon la revendication 1, caractérisé en ce que l'on utilise en tant que base aminée organique la pyridine.